# EUROPEAN PATENT APPLICATION

(11) **EP 4 830 823 A1**
(43) Date of publication of application: **28.10.2026**
(21) Application number: 24907682.9
(22) Date of filing: 05.09.2024
(51) Int. Cl.: B01J 8/18, B01J 19/00, C07C 2/08, C07C 11/02, C07C 11/107

(54) **BUBBLE COLUMN REACTOR AND APPARATUS FOR PRODUCING OLIGOMERS COMPRISING SAME**

(30) Priority: 21.12.2023 KR 20230188410
(71) Applicant: LG CHEM, LTD., Seoul 07336 (KR)
(72) Inventor: SONG, Jong Hun, Daejeon 34122 (KR); LEE, Jeong Seok, Daejeon 34122 (KR); BAEK, Se Won, Daejeon 34122 (KR); HWANG, Moon Sub, Daejeon 34122 (KR); PAK, Geun Tae, Daejeon 34122 (KR)
(74) Representative: Hoffmann Eitle
(86) International application number: PCT/KR2024/013417
(87) International publication number: WO 2025/135396

(57) **Abstract**

The present disclosure provides a bubble column reactor including: a liquid region in which a reaction takes place and a gas region located above the liquid region; a partition wall part that divides the liquid region into first and second zones in a longitudinal direction; a liquid level gauge that is connected to the second zone of the liquid region and measures the liquid level of the second zone; and a downstream pipe that is provided on a side surface of the second zone in the liquid region and discharges a liquid stream, in which the partition wall part is provided in the liquid region so that one end thereof is located at a boundary between the liquid region and the gas region to store the liquid stream of the first zone overflowing above the partition wall part in the second zone, and an apparatus for preparing an oligomer including the same.

## Description

### [Technical Field]

### CROSS-REFERENCE TO RELATED APPLICATION(S)

This application claims priority to and the benefit of Korean Patent Application No. 10-2023-0188410 filed on December 21, 2023, which is hereby incorporated by reference in their entirety.

### Technical Field

The present disclosure relates to a bubble column reactor and an apparatus for preparing an oligomer including the same, and more particularly, to a bubble column reactor capable of preventing pipe clogging by minimizing gas hole up accompanying in a liquid stream including a reaction product, and an apparatus for preparing an oligomer including the same.

### [Background Art]

Alpha-olefins are an important material used in comonomers, detergents, lubricants, plasticizers, and the like, and are commercially widely used. Among them, 1-hexene and 1-octene have been widely used as comonomers for controlling a density of polyethylene at the time of preparing linear low-density polyethylene (LLDPE).

These alpha olefins may be prepared by performing an oligomerization reaction in a state where ethylene is dissolved in a solvent in the presence of a catalyst, and the oligomerization reaction may be performed, for example, in a bubble column reactor.

The bubble column reactor causes a raw material gas to flow into a liquid region containing a solvent, a catalyst, etc., through a distribution device (e.g., a sparger, a multi-pipe, a spray nozzle, etc., having a plurality of holes) installed at a lower portion thereof and forming a large amount of gas hole up and to be dispersed while rising in the form of bubble, and performs an oligomerization reaction by mixing the dispersed gas within the liquid region while generating turbulence.

In this case, since the liquid level in the reactor affects the control of reaction conditions such as pressure and temperature, a catalyst residence time, the resulting ethylene conversion amount, a heating value, etc., the liquid level in the reactor should be maintained constant. To this end, it is very important to indicate a liquid height in the reactor.

As the solvent, the ethylene dissolved in the solvent, and the liquid substance (oligomer) generated by conversion from the ethylene exist together in the liquid region where the oligomerization reaction takes place in the reactor, the composition of the liquid changes, so a density of the liquid after the reaction changes compared to the liquid before the reaction. In particular, the density of the entire liquid volume may be greatly reduced by the amount of accompanying gas hole up in the liquid. In this way, the density is affected by the characteristics (temperature, pressure, composition, viscosity, etc.) of the liquid substance in the reactor.

In order to measure the liquid level in the reactor during the preparation of the oligomer, a differential pressure type level transmitter (LT) is usually applied, but the liquid density may change depending on the degree of reaction, and thus, the liquid level may be incorrectly indicated. When the liquid level incorrectly indicated by the differential pressure type LT occurs significantly, it is difficult to maintain the actual liquid level in the reactor constant.

In addition, when a large amount of gas hole up is accompanied in the liquid material produced by the oligomerization reaction and are transferred to a liquid discharge pipe connected to a lower portion of the reactor, a serious clogging phenomenon may occur due to an increase in two-phase in the pipe.

### [Disclosure]

### [Technical Problem]

The present disclosure provides a bubble column reactor capable of preventing pipe clogging by minimizing the accompanying gas hole up in a liquid stream containing a reaction product.

### [Technical Solution]

In one general aspect, a bubble column reactor includes: a liquid region in which a reaction takes place and a gas region located above the liquid region; a partition wall part that divides the liquid region into first and second zones in a longitudinal direction; a liquid level gauge that is connected to the second zone of the liquid region and measures the liquid level of the second zone; and a downstream pipe that is provided on a side surface of the second zone in the liquid region and discharges a liquid stream, in which the partition wall part is provided in the liquid region so that one end thereof is located at a boundary between the liquid region and the gas region to store the liquid stream of the first zone overflowing above the partition wall part in the second zone.

In one general aspect, the present disclosure provides an apparatus for preparing an oligomer including the bubble column reactor.

### [Advantageous Effects]

The bubble column reactor according to the present disclosure is provided with a partition wall part that divides a lower liquid region where a reaction takes place into first and second zones at a height located at a boundary between a gas region and a liquid region, so a liquid stream of the first zone that has risen above the boundary height may be stored in the second zone, and the liquid stream stored in the second zone may minimize a change in density because gas hole up is removed by the partition wall part. Thereafter, a height of the liquid in which gas-liquid separation takes place in the second zone of the liquid region is measured to control the amount of liquid stream discharged, so a liquid level of the reactor may be stably maintained.

In addition, by suppressing the amount of gas hole up in the liquid stream discharged from the second zone of the liquid region, it is possible to smooth a flow of oligomer products in a liquid discharge pipe, prevent a clogging phenomenon, and reduce the discharge of ethylene gas to a downstream stream.

### [BRIEF DESCRIPTION OF THE DRAWINGS]

FIG. 1 is a diagram illustrating a bubble column reactor according to an embodiment of the present disclosure.
FIGS. 2 and 3 are diagrams illustrating a bubble column reactor used in Comparative Examples.

### [Best Mode]

Terms and words used in the present specification and claims are not to be construed as a general or dictionary meaning, and are to be construed as meaning and concepts meeting the technical spirit of the present disclosure based on a principle that the inventors may appropriately define the concepts of terms in order to describe their own inventions in the best mode.

A term "including" or "containing" used herein concretely indicates specific properties, regions, integer numbers, steps, operations, elements, or components, and is not to exclude presence or addition of other specific properties, regions, integer numbers, steps, operations, elements, components, or a group thereof.

The term 'stream' used herein may mean a flow of a fluid in a process, and may also mean a fluid itself flowing in a pipe. Specifically, the stream may mean both the fluid itself and the flow of the fluid flowing within the pipe connecting each device. In addition, the fluid may include any at least one components of a gas, a liquid, and a solid.

Unless otherwise specified, the term 'upper portion' as used herein means a point at a height of 0 to 50% downward from a top of a device, and may specifically mean the top (top of column). In addition, the term 'lower portion' means a point at a height of 50 to 100% downward from the top of the device, and may specifically mean the bottom (bottom of column).

In addition, the "pressure" mentioned herein means a gauge pressure measured based on atmospheric pressure.

Hereinafter, the present disclosure will be described in detail with reference to the accompanying drawings.

An embodiment of the present disclosure relates to a bubble column reactor for performing an oligomerization reaction, etc.

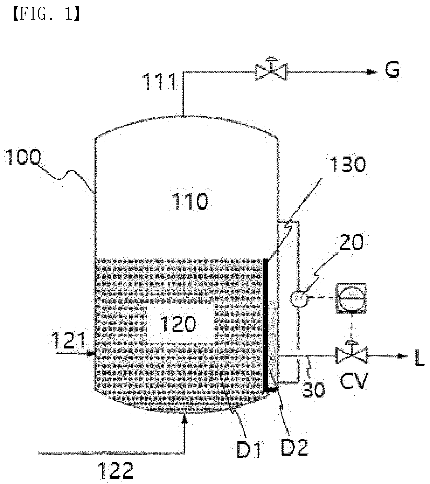

Referring to FIG. 1, a bubble column reactor 100 according to the present disclosure includes an upper gas region 110 and a lower liquid region 120 where a reaction takes place, and the liquid region 120 is divided into a first zone D1 and a second zone D2 in a longitudinal direction by a partition wall part 130, and a liquid level gauge 20 and a downstream pipe 30 for discharging a liquid stream may be connected to the second zone D2.

The bubble column reactor 100 receives an ethylene gas and a solvent from the liquid region 120, specifically, the first zone D1, and performs an oligomerization reaction. For example, the ethylene gas is supplied from a gas inlet pipe 122 to the liquid region 120 to which a solvent is supplied together with a catalyst through a solvent inlet pipe 121, and the ethylene gas may be dispersed while rising in the form of bubbles through a distribution device (e.g., a sparger, a multi-pipe, a spray nozzle, etc., having a plurality of holes) installed at a lower portion of the liquid region 120 and forming a large amount of gas holes up to be mixed within the liquid region while generating turbulence, so the oligomerization reaction may be performed.

The oligomerization reaction may refer to a reaction in which the monomers are oligomerized. Depending on the number of monomers that are oligomerized, the oligomerization reaction is called trimerization or tetramerization, and is collectively called multimerization. The alpha olefins such as 1-hexene and 1-octene may be prepared, for example, through trimerization or tetramerization of ethylene.

The ethylene gas supplied to the bubble column reactor may be a stream containing ethylene (C2) separated from naphtha thermal cracking.

The solvent for dissolving the ethylene gas may include one or more selected from the group consisting of n-pentane, n-hexane, n-heptane, cyclohexane, methyl cyclohexane, octane, cyclooctane, decane, dodecane, benzene, xylene, 1,3,5-trimethylbenzene, toluene, ethylbenzene, chlorobenzene, dichlorobenzene, and trichlorobenzene. In some cases, a mixture of two or more of those described above may be used as the solvent. Therefore, the ethylene gas may be liquefied at a higher temperature, and a dissolution rate at which the ethylene gas is dissolved in the solvent may be improved.

In the oligomerization reaction of the ethylene, a compound containing a transition metal may be used as a catalyst to promote reaction activity. For example, the catalyst may be a compound containing one or more selected from the group consisting of chromium(III) acetylacetonate, chromium(III) chloride tetrahydrofuran, chromium(III) 2-ethylhexanoate, chromium(III) tris(2,2,6,6-tetramethyl-3,5-heptanedionate), chromium (III) benzoylacetonate, chromium (III) hexafluoro-2,4-pentanedionate, chromium (III) acetate hydroxide, chromium (III) acetate, chromium(III) butyrate, chromium(III) pentanoate, chromium(III) laurate, and chromium(III) stearate.

In addition, a cocatalyst may be additionally used to increase the activity of the above catalyst. The cocatalyst may include one or more selected from the group consisting of, for example, trimethyl aluminum, triethyl aluminum, triisopropyl aluminum, triisobutyl aluminum, ethylaluminum sesquichloride, diethylaluminum chloride, ethyl aluminum dichloride, methylaluminoxane, modified methylaluminoxane, and borate.

The oligomerization reaction may be operated under conditions commonly applied in the relevant field, and may be performed at a temperature of 30°C to 150°C or 50°C to 120°C and a pressure of 20 bar to 65 bar or 20 bar to 40 bar, for example.

When this oligomerization reaction is performed, a liquid stream containing an oligomer product polymerized with ethylene, a by-product, a solvent, an unreacted gas dissolved in the solvent, etc., may exist in the lower liquid region 120 of the bubble column reactor 100, and the unreacted ethylene gas that is not dissolved in the solvent and thus does not participate in the oligomerization reaction may rise to the upper gas region 110.

The liquid stream existing in the liquid region 120 of the bubble column reactor may have a density changing depending on the amount of liquid oligomer product generated by conversion from the ethylene and the amount of gas hole up derived from the unreacted gas dissolved in the solvent, and when an incorrect instruction occurs during the measurement of the liquid level due to the change in density, it may be difficult to maintain the liquid level in the reactor stably.

To overcome these problems, in the present disclosure, the partition wall part 130 that acts as a weir is installed in the liquid region 120 of the bubble column reactor in a longitudinal direction, and the liquid level after gas-liquid separation is measured in the space where the liquid stream that has passed the partition wall part is stored.

Specifically, the partition wall part 130 may be provided so that one end is located at the boundary between the liquid region 120 and the gas region 110 while dividing the liquid region 120 into the first zone D1 and the second zone D2 in the longitudinal direction, and there is no special limitation as long as it has a form that may confine the liquid stream in the liquid region 120.

For example, as illustrated in FIG. 1, the partition wall part 130 may confine the liquid stream in a form that it has a vertical wall in the longitudinal direction and a horizontal plate that extends horizontally from the lower end of the vertical wall and contacts a side surface of the second liquid region, and the horizontal plate may be adjacent to or spaced apart from a lowest surface of the liquid region 120.

As another example, the partition wall part 130 may confine the liquid stream in the form of the vertical wall extending upward from the lowermost surface of the liquid region 120. In this case, in the liquid region 120 divided by the partition wall part 130, the lower portion of the first zone D1 may be configured to open the distribution device (e.g., a sparger, a multi-pipe, a spray nozzle, etc., having a plurality of holes) to disperse the ethylene gas supplied from the gas inlet pipe 122 to the first zone D1, while the lower portion of the second zone D2 may be configured to be clogged to prevent the ethylene gas from passing.

As another example, the partition wall part 130 may be configured in the form of a vertical pipe having a donut-shaped rim and a clogged lower portion to confine the liquid stream, and this donut-shaped partition wall part may be provided at various locations, such as a center or edge of the liquid region 120.

Meanwhile, the first zone D1 and the second zone D2 of the liquid region divided by the partition wall part 130 may have an area ratio of 100:1 to 5:1. When the area ratio is out of the above range, that is, when the area of the second zone D1 is less than 1% of the area of the first zone D1, the space for controlling the height of the liquid is insufficient, so that a residence time of the liquid in the second zone D2 decreases, making it difficult to accurately control the liquid level. When the area of the second zone D1 exceeds 20% of the area of the first zone D1, the residence time of the liquid in the second zone D2 increases excessively, so that not only additional side reactions are performed, but also the size of the entire reactor should increase, which may increase the equipment cost.

Through this structure, while the oligomerization reaction is performed in the liquid region 120 of the bubble column reactor, the liquid stream of the first zone D1 that has risen above the boundary height of the gas region 110 and the liquid region 120 may overflow above the partition wall part 130 and be stored in the second zone D2. That is, due to the difference in boiling points between the liquid and the gas at the boundary between the liquid and the gas, the ethylene gas passed through the first zone D1 of the liquid region 120 in the form of bubbles, passed through the gas region 110, and was then discharged through the upper pipe 111, while the liquid is transferred to the second zone D2 through the partition wall part 130. The liquid stored in the second zone D2 may suppress the accompanying of the gas hole up.

In this way, since the liquid stream stored in the second zone D2 of the liquid region is in a state where the gas hole up is removed by the partition wall part 130 and the gas-liquid separation is achieved, there is no need to install a separate gas-liquid separator outside the reactor.

The existing technology produces olefin by oligomerization of ethylene in the liquid region of the bubble column reactor and separates the unreacted gas by discharging a liquid stream containing the olefin from the reactor and transferring the discharged liquid stream to an external gas-liquid separator, and had a problem in that the temperature decreased according to the residence time of the liquid during this process and by-products such as polymer were precipitated. In order to prevent this, the existing technology requires an additional device to maintain the temperature of the gas-liquid separator, but the present disclosure may perform the gas-liquid separation inside the bubble column reactor to prevent the precipitation of by-products due to the temperature drop and does not require an additional device, so it is advantageous in terms of facility cost and maintenance cost.

The liquid stream from which gas is separated in the second zone D2 of the liquid region 120 is measured by the liquid level gauge 20 connected to the second zone D2.

The above-mentioned liquid level gauge may be a differential pressure type level transmitter (LT) commonly used in the relevant field, but is not limited thereto.

The liquid stored in the second zone D2 of the above liquid region 120 does not have a large change in density because the gas hole up is removed and the composition of the liquid does not change. Therefore, unlike the first zone D1 where the liquid composition changes, the possibility of occurrence of the incorrect instruction is low when measuring the liquid level in the second zone D2.

Therefore, the height of the liquid in which the change in density is suppressed is measured using the liquid level gauge 20 connected to the second zone D2, and the liquid stream in the second zone D2 is discharged according to the signal of the liquid level gauge 20, so the liquid level of the bubble column reactor may be stably maintained.

The liquid stream in the second zone D2 of the liquid region 120 may be discharged through the downstream pipe 30 connected to the second zone D2. In this case, the amount of liquid stream discharged may be controlled by controlling the opening and closing of the control valve CV provided in the downstream pipe 30 according to the signal of the liquid level gauge 20.

In addition, by minimizing the amount of gas hole up in the liquid stream discharged from the second zone D2 of the liquid region 120, it is possible to smooth a flow of oligomer products in the pipe, prevent the clogging phenomenon, and reduce the discharge of ethylene gas to the downstream stream.

The present disclosure further provides an apparatus for preparing an oligomer including the bubble column reactor.

The liquid stream discharged through the downstream pipe 30 in the bubble column reactor may be supplied to a separation tower (not illustrated), and the unreacted gas, solvent, etc., may be additionally separated in the separation tower to obtain the purified oligomer product.

### [Mode for Invention]

Hereinafter, the present disclosure will be described in more detail through Examples. However, the following Examples are provided in order to exemplify the present disclosure, it is apparent to those skilled in the art that various modifications and alterations may be made without departing from the scope and spirit of the present disclosure, and the scope of the present disclosure is not limited to these Examples.

### Comparative Example 1:

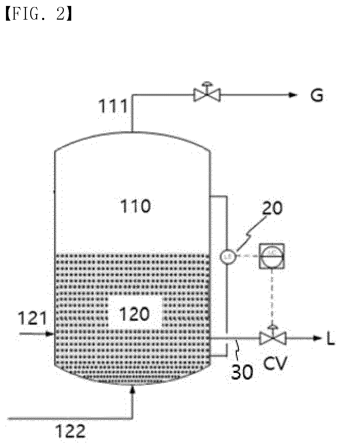

As illustrated in FIG. 2 in the bubble column reactor including the upper gas region 110, the lower liquid region 120, the liquid level gauge 20, and the downstream pipe 30, the solvent and catalyst were supplied to the lower liquid region 120 through the solvent inlet pipe 121, and ethylene gas was supplied through the gas inlet pipe 122 to perform the oligomerization reaction.

The height of the liquid stream containing the oligomer product obtained by the reaction, the solvent, and the unreacted ethylene gas dissolved in the solvent was measured using the differential pressure type level transmitter (LT) as the liquid level gauge 20, and the liquid stream was discharged through the downstream pipe 30 of the reactor. In this case, the amount of liquid stream discharged was controlled using the control valve CV according to the liquid level measurement value to maintain the liquid level in the reactor. Meanwhile, the gas stream containing the unreacted ethylene gas was discharged through the upper pipe 111 of the gas region 110.

### Comparative Example 2:

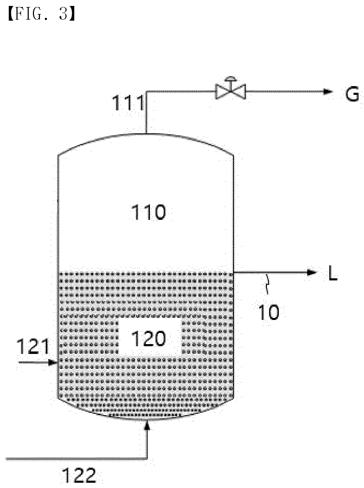

As illustrated in FIG. 3, in the bubble column reactor including the upper gas region 110, the lower liquid region 120, and the overflow pipe 20 connected to the boundary position of the gas region and the liquid region, the solvent and catalyst were supplied to the lower liquid region 120 through the solvent inlet pipe 121, and ethylene gas was supplied through the gas inlet pipe 122 to perform the oligomerization reaction.

The liquid stream containing the oligomer product obtained by the reaction, the solvent, and the unreacted ethylene gas dissolved in the solvent was discharged through the overflow pipe 10 when it has risen above the boundary position between the gas region 110 and the liquid region 120, thereby maintaining the liquid level in the reactor. Meanwhile, the gas stream containing the unreacted ethylene gas was discharged through the upper pipe 111 of the gas region 110.

### Example 1:

As illustrated in FIG. 1, in the bubble column reactor including the upper gas region 110, the lower liquid region 120, the partition wall part 130 that are provided at the height located at the boundary between the gas region and the liquid region and divides the liquid region 120 into the first zone D1 and the second zone D2, the liquid level gauge 20, and the downstream pipe 30, the solvent and catalyst were supplied to the first zone D1 of the lower liquid region 120 through the solvent inlet pipe 121 and the ethylene gas was supplied through the gas inlet pipe 122, thereby performing the oligomerization reaction.

During the reaction, the liquid stream of the first zone D1, which has risen the boundary height between the gas region 110 and the liquid region 120, overflowed above the partition wall part 130 and was stored in the second zone D2. In this case, due to the difference in boiling points between the liquid and the gas at the boundary between the liquid and the gas, the ethylene gas passed through the first zone D1 of the liquid region 120 in the form of bubbles, passed through the gas region 110, and was then discharged through the upper pipe 111, while the liquid was transferred to the second zone D2 through the partition wall part 130 while the gas hole up is removed.

Thereafter, in the second zone D2 of the liquid region 120, the liquid level of the liquid stream in which the gas hole up is removed, that is, the gas is separated, was measured using the differential pressure LT as the liquid level gauge 20 connected to the second zone, and the liquid stream was discharged through the downstream pipe 30 provided in the second zone D2. In this case, the amount of liquid stream discharged was controlled using the control valve CV provided in the liquid discharge pipe 30 according to the liquid level measurement value to maintain the liquid level in the reactor. Meanwhile, the gas stream containing the unreacted ethylene gas was discharged through the upper pipe 111 of the gas region 110.

The results of the liquid level measurement and the liquid maintenance of the reactor according to the above examples and comparative examples were shown in Table 1 below.

**[Table 1]**

| | Liquid discharge location | Liquid level measurement target | Liquid maintenance result |
|---|---|---|---|
| Comparative Example 1 | Downstream pipe | Entire liquid region | Increase in liquid level of reactor |
| Comparative Example 2 | Overflow pipe | - | Maintain liquid level of reactor /discharge large amount of gas through overflow pipe |
| Exampl | Downstrea | Second zone D2 | Maintain liquid level of |
| e 1 | m pipe | of liquid region divided by partition wall part | reactor |

As can be seen in the above Table 1, in Example 1, the partition wall part that divides the first zone D1 where the reaction takes place in the lower liquid region 120 and the second zone D2 where the liquid stream from which the gas hole up is removed is stored is provided at the height located at the boundary of the gas region and the liquid region to store the liquid stream in the first zone, which has risen above the boundary height, in the second zone while performing the gas-liquid separation by the partition wall part and then measure the liquid height, and as a result, there is almost no change in density due to the gas hole up in the liquid in the second zone, so the incorrect instruction of the differential pressure LT is minimized. Therefore, the amount of liquid discharged was controlled through the liquid level control valve CV connected to the differential pressure LT, and the liquid level of the reactor was finally maintained constant.

In addition, by minimizing the amount of gas hole up in the liquid stream in the second zone D2 of the liquid region 120, it is possible to smooth the flow of oligomer products in the downstream pipe, prevent the clogging phenomenon, and reduce the amount of ethylene gas to be recovered from the pipe.

In contrast, in Comparative Example 1, the incorrect instruction to decrease the LT indication value at the liquid level having the same height occurred due to the direct measurement of the liquid accompanied by the gas hole up in the reactor 100. This is because the ethylene gas supplied from the lower portion of the reactor was included in the liquid as the gas hole up in the form of bubble as it passed through the liquid, and as the oligomer products were included after the reaction and the gas hold-up space increased compared to the liquid where only the solvent existed at the beginning of the reaction, the density of the liquid decreased, so that the differential pressure decreased even if the liquid level existed at the same height, and the LT indication value was displayed as the decrease in the liquid level. The control valve is closed to control the liquid level by the incorrect instruction, so the actual liquid level in the reactor finally rises. Due to this increase in the liquid level in the reactor, the catalyst residence time in the reactor became longer, there were limits to the control of the amount of products and the heating value, the clogging phenomenon due to polymer occurred in the pipe through which the liquid stream is discharged, and the amount of ethylene to be recovered from the pipe increased.

Meanwhile, in Comparative Example 2, as a result of controlling the liquid level in the reactor 100 by discharging the liquid that has risen above the boundary height through the overflow pipe 10 at the boundary between the gas region and the liquid region including the gas hole up without separately measuring the liquid level in the reactor 100, a large amount of gas stream containing the unreacted ethylene of the gas region was discharged together with the liquid stream through the overflow pipe 10. As a result, it is difficult to predict the content of ethylene discharged through the overflow pipe, and the clogging phenomenon occurred due to the increase in the two-phase of liquid-gas within the pipe.

### [Detailed Description of Main Elements]

100: Bubble column reactor
110: Gas region
111: Gas stream discharge pipe
120: Liquid region (D1: first zone, D2: second zone)
121: Solvent inlet pipe
122: Gas inlet pipe
130: Partition wall part
10: Overflow pipe
20: Liquid level gauge
30: Downstream pipe
CV: Liquid level control valve

## Claims

1. A bubble column reactor, comprising:
a liquid region in which a reaction takes place and a gas region located above the liquid region;
a partition wall part that divides the liquid region into first and second zones in a longitudinal direction;
a liquid level gauge that is connected to the second zone of the liquid region; and
a downstream pipe that is provided on a side surface of the second zone in the liquid region and discharges a liquid stream,
wherein the partition wall part is provided in the liquid region so that one end thereof is located at a boundary between the liquid region and the gas region to store the liquid stream of the first zone overflowing above the partition wall part in the second zone.

2. The bubble column reactor of claim 1, wherein the liquid region receives an ethylene gas and a solvent to perform an oligomerization reaction.

3. The bubble column reactor of claim 1, further comprising:
an upper pipe that discharges a gas stream containing an unreacted gas from the gas region.

4. The bubble column reactor of claim 1, wherein the partition wall part is provided with a vertical wall in a longitudinal direction and a horizontal plate that extends transversely from a lower end of the vertical wall and contacts a side surface of the second liquid region.

5. The bubble column reactor of claim 1, wherein the horizontal plate of the partition wall part is adjacent to or spaced apart from a lowest surface of the liquid region.

6. The bubble column reactor of claim 1, wherein the partition wall part is in a form of a vertical wall extending upward from a lowest surface of the liquid region.

7. The bubble column reactor of claim 1, wherein the partition wall part is in a form of a vertical pipe that has a donut-shaped rim and has a lower portion clogged.

8. The bubble column reactor of claim 1, wherein the first zone and the second zone in the liquid region are divided in an area ratio of 100:1 to 20:1.

9. The bubble column reactor of claim 1, wherein the liquid stream stored in the second zone of the liquid region is subjected to gas-liquid separation.

10. The bubble column reactor of claim 1, wherein the liquid level gauge measures a liquid level of the liquid stream from which gas-liquid is separated in the second zone of the liquid region.

11. The bubble column reactor of claim 1, wherein a liquid discharge pipe connected to a lower portion of the second zone in the liquid region includes a valve that controls the amount of liquid discharged according to a signal of the liquid level gauge.

12. An apparatus for preparing an oligomer including a bubble column reactor of claim 1.
